# EUROPÄISCHE PATENTSCHRIFT

(11) **EP 1 176 912 B1**
(45) Veröffentlichungstag und Bekanntmachung des Hinweises auf die Patenterteilung: **17.12.2003**
(21) Anmeldenummer: 00938608.7
(22) Anmeldetag: 20.04.2000
(51) Int. Cl.: A61B 17/02

(54) **RETRAKTOR ZUR VERWENDUNG IN DER ENDOSKOPISCHEN CHIRURGIE**
RETRACTOR FOR USE IN ENDOSCOPIC SURGERY
RETRACTEUR UTILISE EN CHIRURGIE ENDOSCOPIQUE

(30) Priorität: 06.05.1999 DE 19920869
(43) Veröffentlichungstag der Anmeldung: 06.02.2002
(73) Patentinhaber: Karl Storz GmbH & Co. KG, 78532 Tuttlingen (DE)
(72) Erfinder: CUSCHIERI, Alfred, St. Andrews Fife KY16 9TY (GB); FRANK, Timothy Graham, Wormit, Fife KY16 9TY (GB)
(74) Vertreter: Hofmeister, Frank Horst
(86) Internationale Anmeldenummer: EP0003604
(87) Internationale Veröffentlichungsnummer: WO00067642

(56) Entgegenhaltungen:
- WO-A-93/13713
- WO-A-93/22973
- WO-A-97/23158
- US-A- 5 382 231
- US-A- 5 522 788
- US-A- 5 624 381
- US-A- 5 787 897

## Beschreibung

Die Erfindung betrifft einen Retraktor zur Verwendung in der endoskopischen Chirurgie, mit einem Schaft, an dessen proximalem Ende ein Handstück angeordnet ist und dessen distales Ende aus einer gestreckten geraden Ausgangsposition verstellbar ist, wobei das verstellbare distale Ende aus mehreren gegeneinander verschwenkbaren Gliederelementen besteht, die in eine ringförmige Struktur verstellbar sind und wobei diese ringförmige Struktur gegenüber dem starren Teil des Schaft abwinkelbar ist.

Retraktoren werden in der endoskopischen Chirurgie dazu verwendet, um bei der Operation unter endoskopischer Sicht Organe und dgl. Körperteile, die nicht zu behandeln sind, aus dem Operationsgebiet herauszuhalten.

Aus der WO-A-93/13713 ist gattungsgemäßer Retraktor bekannt. Das distale Ende dieses bekannten Retraktors besteht aus mehreren gegeneinander verschwenkbaren Gliederelementen, die in ihrem Inneren über einen Seilzug miteinander verbunden sind, der wiederum an den Handgriff des Retraktors angeschlossen ist. Beim Festziehen einer am Handgriff angeordneten Mutter wird der Seilzug gespannt und werden die einzelnen Gliederelemente gegeneinander gezogen. Da die einander zugewandten Stirnflächen der einzelnen Gliederelemente zumindest einseitig abgewinkelt ausgebildet sind, bewirkt diese gegenseitige Verspannung, daß die Gliederelemente aus ihrer ebenen Ausgangslage ausgelenkt werden. Im äußersten Fall ist es dabei möglich, die Gliederelemente des distalen Endes so weit abzuwinkeln, daß das distale Ende eine offene Ringstruktur bildet. Die Ausbildung einer offenen Ringstruktur hat den Nachteil, daß diese offene Struktur nicht in sich stabil und tragfähig ist, so daß keine großen Kräfte auf das abzuhaltende Gewebe ausgeübt werden können, ohne daß die Gefahr besteht, daß die offene Ringstruktur nachgibt und das abzuhaltende Gewebe wieder freigibt.

Das Abwinkeln des verstellten distalen Endes gegenüber dem Schaft ist bei dem bekannten Retraktor nur um 90° möglich, da das Abwinkeln auch über den im Inneren der Gliederelemente angeordneten Seilzug erfolgt. Wenn das distale Ende die Ringstruktur einnehmen soll, muß über die am Handgriff angeordnete Mutter der Seilzug vollständig angezogen werden. Dieses vollständige Anziehen des Seilzugs bewirkt dann aber gleichzeitig, daß die Ringstruktur auch vollständig, das heißt um 90° gegenüber dem Schaft abgewinkelt wird. Jede Zwischenstellung der Abwinklung bedeutet, daß die Ringstruktur nicht vollständig ausgeformt ist und das distale Ende des Retraktors noch weniger dazu geeignet ist, Gewebe und/oder Organe aus dem Operationsgebiet zu entfernen

Darüber hinaus werden in der Praxis üblicherweise Fächerretraktoren verwendet. Ein solcher Fächerrtraktor ist beispielsweise aus der US-PS 5 307 805 bekannt. Die Fächerretraktoren weisen den Nachteil auf, daß beim Zusammenschieben des Retraktors die Gefahr besteht, daß die mit dem Retraktor behandelten Körperteile durch Quetschungen verletzt werden.

Ein weiterer Retraktor ist beispielsweise aus der DE 693 10 345 T2 bekannt. Diese Retraktoren werden im gestreckten geraden Zustand in den Körper, beispielsweise den Bauchraum eingeführt und erst dort durch Drehen des Handstücks verstellt. Durch die Verwendung der verstellbaren Gliederelemente kann es zu Quetschungen an zu behandelnden Körperteilen kommen. Darüber hinaus sind diese bekannten Retraktoren insbesondere im Übergangsbereich Schaft/Handstück nur schlecht zu reinigen.

Ausgehend von diesem Stand der Technik liegt der Erfindung die Aufgabe zugrunde, einen Retraktor zu schaffen, der einerseits einen atraumatischen Gebrauch gewährleistet und mit dem andererseits sicher und stabil Gewebe und/oder Organe aus dem Operationsgebiet fortgehalten werden können.

Die Lösung dieser Aufgabenstellung ist erfindungsgemäß dadurch gekennzeichnet, daß das distale Ende des Schaftes in eine geschlossene Ringstruktur verstellbar ist, wobei am vorderen freien Ende des vordersten Gliederelements ein Fixierelement angeordnet ist, über das zum Ausbilden der geschlossenen Ringstruktur das vorderste Gliederelement am Schaft festlegbar ist, und daß die geschlossene Ringstruktur gegenüber dem starren Teil des Schaftes stufenlos um bis zu 90° abwinkelbar ist.

Dadurch, daß bei dem erfindungsgemäß ausgebildeten Retraktor das Verstellen hin zu einer geschlossenen Ringstruktur erfolgt, können Quetschverletzungen, wie sie bei den bekannten Retraktoren auftreten können, ausgeschlossen werden. Das Ausbilden der geschlossenen Ringstruktur mit dem am Schaft festgelegten vordersten Gliederelement hat darüber hinaus den Vorteil, daß das verstellte distale Ende in sich stabil und tragfähig ist. Die Einsetzbarkeit des erfindungsgemäßen Retraktors wird weiterhin dadurch erhöht, daß die Ringstruktur gegenüber dem Schaft stufenlos um bis zu 90° abwinkelbar ist.

Gemäß einer ersten Ausführungsform der Erfindung erfogt das Verstellen des distalen Endes des Schaftes unter endoskopischer Sicht mittels eines Werkzeugs. Diese Ausführungsform zeichnet sich durch ihren einfachen Aufbau auf, da auf einen eigenen Antrieb zum Verstellen des distalen Endes des Retraktorschaftes verzichtet wird.

Um das Öffnen und /oder Schließen der Ringstruktur mittels eines Werkzeugs zu erleichtern, sind am vordersten Gliederelement Ausnehmungen ausgebildet, in zum Verstellen das Werkzeug , insbesondere eine Zange, eingreifen kann.

Gemäß einer zweiten Ausführungsform der Erfindung ist das distale Ende des Retraktorschaftes mittels eines innen- oder außenliegenden Bowdenzuges verstellbar.

Gemäß einer ersten praktischen Ausführungsform ist das Fixierelement als Rastelement ausgebildet, das in einer Rastaufnahme des ersten, mit dem starren Teil des Schaftes verbundenen Gliederelements festlegbar ist. Durch diese feste Verrastung miteinander ist es möglich, über die geschlossene Ringstruktur relativ große Kräfte auszuüben.

Gemäß einer alternativen Ausführungsform der Erfindung ist das Fixierelement als Magnet ausgebildet.

Gemäß einer praktischen Ausführungsform der Erfindung ist die Ringstruktur über ein Gelenk zwischen dem starren Schaftteil und dem ersten Gliederelement in beide Richtungen aus der Ebene der Ringstruktur um mindestens bis zu 90° gegenüber dem Schaft abwinkelbar.

Um das Sterilisieren des erfindungsgemäßen Retraktors zu erleichtern, wird weiterhin vorgeschlagen, daß das Handstück und der Schaft auswechselbar über ein Adapterstück miteinander verbunden sind, so daß die Teile einzeln gereinigt werden können.

Eine besonders einfache Montage von Schaft und mit dem Handstück verbundenen Adapterstück kann dabei dadurch erzielt werden, daß Adapterstück und Schaft miteinander verrastet sind.

Zum Festlegen des Schaftes am Adapterstück wird vorgeschlagen, daß das Adapterstück eine axiale Bohrung aufweist, in der ein von außen über einen Knopf betätigbares Rastelement zum Festlegen des Schaftes angeordnet ist. Zum Verbinden von Schaft und Handstück ist es somit lediglich notwendig, das proximale freie Ende des Schaftes in die axiale Bohrung des mit dem Handstück verbundenen Adapterstücks einzuschieben, bis das im Adapterstück angeordnete Rastelement den Schaft verrastend fixiert.

Um eine sichere Verbindung zwischen Schaft und Adapterstück über das Rastelement des Adapterstücks zu gewährleisten und auch das Lösen dieser Verbindung durch die Betätigung des auf der Außenseite des Adapterstücks angeordneten Knopfs zu ermöglichen, ist das Rastelement in der Raststellung federbelastet ausgebildet.

Die Handhabung des erfindungsgemäßen Retraktors für das Operationsteam kann dadurch erleichtert werden, daß das Gewicht des Handstücks so bemessen ist, daß das Handstück als Gegengewicht zu dem mittels der Ringstruktur ergriffenen Körperteils dient.

Weitere Merkmale und Vorteile der Erfindung ergeben sich aus der nachfolgenden Beschreibung der zugehörigen Zeichnung, in der zwei Ausführungsbeispiele eines erfindungsgemäßen Retraktors und ein Ausführungsbeispiel für eine Einführhilfe dargestellt sind. In der Zeichnung zeigt:
- Fig. 1a: eine perspektivische Ansicht eines erfindungsgemäßen Retraktors ohne Handstück mit teilweise verstelltem distalem Ende und einem Fixierelement gemäß einer ersten Ausführungsform;
- Fig. 1b: eine Seitenansicht einer zweiten Ausführungsform des Fixierelements für einen Retraktor gemäß Fig. 1 a;
- Fig. 1c: eine um 90° gedrehte Seitenansicht des Fixierelements gemäß Fig. 1 b;
- Fig. 2: eine perspektivische Explosionszeichnung eines Adapterstücks;
- Fig. 3: einen Längsschnitt durch das Adapterstück gemäß Fig. 2 im zusammengebauten Zustand;
- Fig. 4: eine perspektivische Explosionszeichnung von Handstück und Adapterstück und
- Fig. 5: eine perspektivische Ansicht einer Einführhilfe.

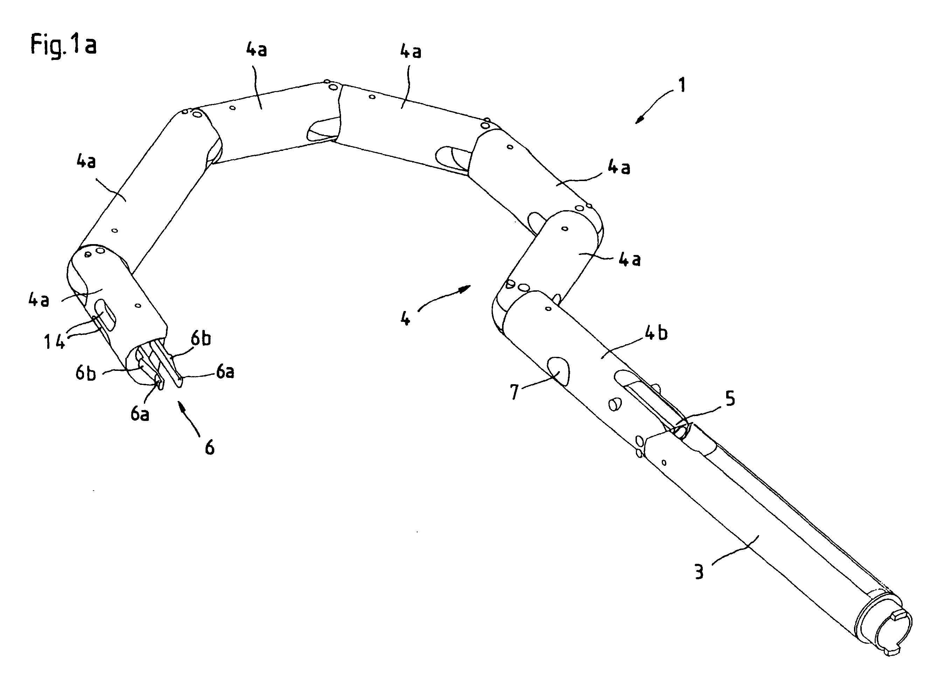

Fig. 1a zeigt einen Schaft 1 eines Retraktors, jedoch ohne ein noch näher zu beschreibendes Handstück 2, welches im montierten Zustand des Retraktors am proximalen Ende des Schaftes 1 angeordnet ist.

Der dargestellte Schaft 1 besteht aus einem mit dem Handstück 2 zu verbindenden starren Teil 3 und am distalen Ende aus sieben gelenkig mit dem starren Teil 3 verbundenen Gliederelementen 4. Während die vom freien distalen Ende des Schaftes 1 aus gesehen vorderen sechs Gliederelemente 4a in der selben Ebene zueinander verschwenkbar sind, ist das mit dem starren Teil 3 des Schaftes 1 verbundene Gliederelement 4b über ein Gelenk 5 aus der Ebene der Gliederelemente 4a stufenlos um bis zu 90° gegenüber dem starren Teil 3 des Schaftes 1 abwinkelbar.

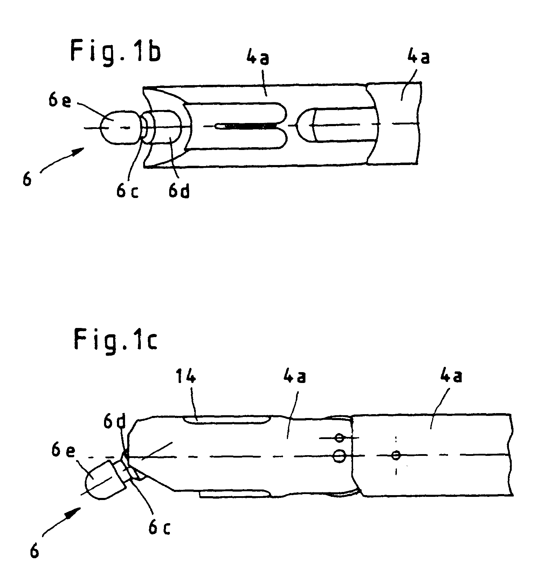

Zur Ausbildung einer geschlossenen Ringstruktur mittels der Gliederelemente 4a ist am vorderen freien Ende des ersten Gliederelements 4a ein Rastelement 6 angeordnet, welches in einer Rastaufnahme 7 festlegbar ist, die in dem mit dem starren Teil 3 verbundenen Gliederelement 4b ausgebildet ist. Beim in Fig. 1 a dargestellten ersten Ausführungsbeispiel besteht das Rastelement 6 aus zwei parallelen vom freien Ende des Gliederelements 4a fortweisenden Armen 6a, an denen jeweils ein Rastvorsprung 6b ausgebildet ist, um bei geschlossener Ringstruktur der Gliederelemente 4a den Rand der Rastaufnahme 7 zu hintergreifen.

Bei der in Fig. 1b und 1c dargestellten zweiten Ausführungsform ist das Fixierelement zum Festlegen des vordersten Gliederelements 4a am Schaft1 wiederum als Rastelement 6 ausgebildet. In diesem Fall wird das Rastelement 6 von einem mit einer umlaufenden Ringnut 6c versehenen Stift 6d gebildet, der an seinem freien Ende mit einem halbkugelförmigen Kopf 6e versehen ist. Zur Ausbildung der geschlossenen Ringstruktur wird das Rastelement 6 in die Rastaufnahme 7 eingesteckt, bis eine (nicht dargestellte) in der Rastaufnahme 7 angeordnete und in Axialrichtung wirkende Kugelraste in die umlaufende Ringnut 6c einrastet und so das vorderste Gliederelement 4a fest mit dem Schaft 1 verbindet.

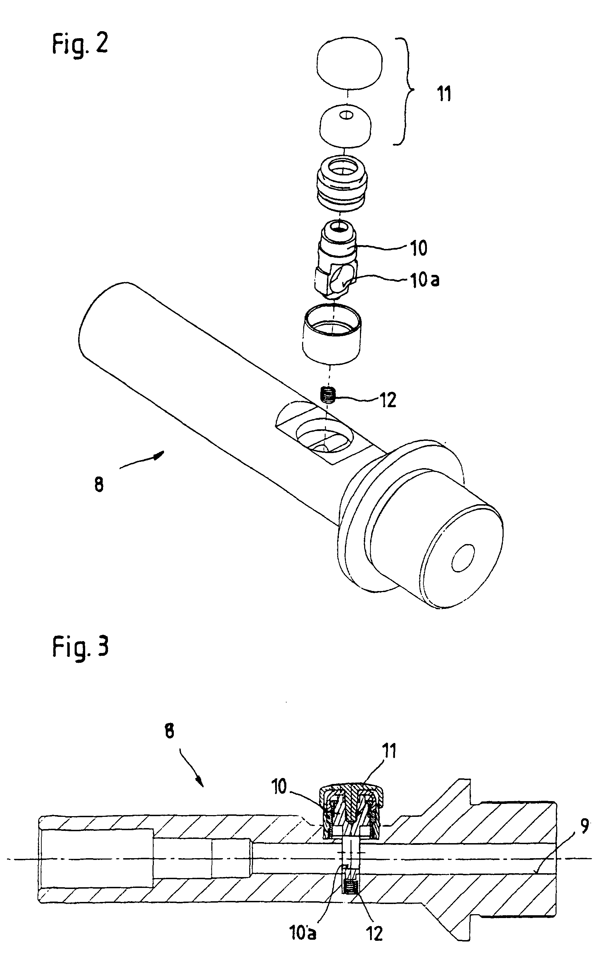

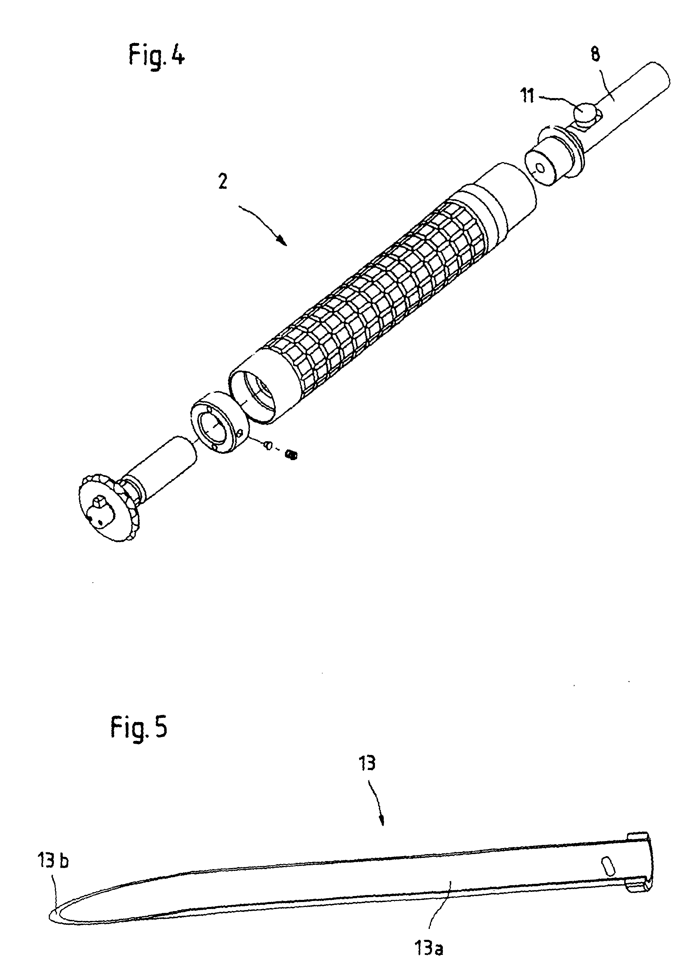

Das in Fig. 4 dargestellte Handstück 2 wird über ein Adapterstück 8 gemäß Fig. 2 und Fig. 3 mit dem proximalen Ende des Schaftes 1 verbunden. Während das Adapterstück 8 in der Regel, beispielsweise durch Verkleben, fest mit dem Handstück 2 verbunden ist, ist die Verbindung zwischen Adapterstück 8 und starrem Teil 3 des Schaftes 1 als auswechselbare Rastverbindung ausgestaltet, so daß der Schaft 1 einfach und schnell zu Reinigungszwecken vom Handstück 2 bzw. Adapterstück 8 gelöst werden kann.

Wie aus Fig. 2 und 3 ersichtlich, weist das Adapterstück 8 eine axiale Durchgangsbohrung 9 auf, in der ein von außen betätigbares Rastelement 10 angeordnet ist. Zum Festlegen des Schaftes 1 weist das Rastelement 10 eine Aufnahmebohrung 10a auf. Das Betätigen der Verriegelung erfolgt von der Außenseite des Adapterstückes her mittels eines Knopfs 11, über den das Rastelement 10 gegen die Kraft einer Druckfeder 12 so weit in das Adapterstück 8 eindrückbar ist, bis die Aufnahmebohrung 10a mit der Durchgangsbohrung 9 fluchtet, so daß der Schaft 1 in das Adapterstück 8 einsetzbar bzw. aus diesem herausziehbar ist.

Neben der dargestellten Verbindung zwischen Schaft 1 einerseits und Handstück 2 bzw. Adapterstück 8 andererseits über das beschriebene Rastelement 10 sind selbstverständlich alle anderen lösbaren Verbindungen zum Verbinden dieser Bauteile anwendbar, um einen einfach handhabbaren und gut zu reinigenden Retraktor zu schaffen.

Fig. 5 zeigt schließlich eine Einführhilfe 13 zum Einführen eines Retraktors in einen menschlichen oder tierischen Körper. Die Einführhilfe 13 weist einen im Querschnitt im wesentlichen halbkreisförmigen Schaft 13a auf, dessen distales Ende als selbstschneidende Spitze 13b ausgebildet ist.

Das Arbeiten mit dem dargestellten Retraktor geschieht wie folgt:

Zum Einführen des Retraktors in das anderweitig bereits vorbereitete Operationsgebiet, beispielsweise die Bauchhöhle wird die Einführhilfe 13 mit der selbstschneidenden Spitze 13b voran durch das Gewebe gestoßen und anschließend entlang der konkaven Innenseite des Schaftes 13a der Retraktor unter endoskopischer Sichtkontrolle in das Operationsgebiet eingeführt. Sobald der Retraktor in den Körper eingeführt ist, kann die Einführhilfe 13 wieder herausgezogen werden, damit der Retraktor frei beweglich ist.

In der Ausgangsposition, in der der Retraktor in den Körper eingeführt wird, ist dieser gerade gestreckt ausgerichtet, d.h., daß alle Gliederelemente 4 fluchtend mit dem starren Teil 3 des Schaftes 1 ausgerichtet sind. Um mit dem Retraktor Organe oder dgl. Körperteile aus dem Operationsgebiet heraushalten zu können, ist es nach dem Einführen des Retraktors notwendig, dessen distales Ende so zu verformen, daß damit die entsprechenden Körperteile sicher erfaßt und gehalten werden können. Zu diesem Zweck werden bei der dargestellten Ausführungsform unter endoskopischer Sichtkontrolle die Gliederelemente 4a mittels eines Werkzeugs, beispielsweise einer Faßzange, soweit gegeneinander verschwenkt, bis diese eine geschlossene Ringstruktur bilden und das Rastelement 6 in die Rastaufnahme 7 eingreift. Um das Verstellen der Gliederelemente 4a mittels des werkzeugs zu erleichtern, weist das vorderste, das Fixierelement tragende Gliederelement 4a Ausnehmungen 14 auf, in die das Werkzeug zum Öffnen oder Schließen der Ringstruktur eingreifen kann. Mit dieser geschlossenen Ringstruktur können nun nahezu ohne jede Verletzungsgefahr die entsprechenden Körperteile sicher vom Operationsgebiet fortgehalten werden, um Platz und Sicht für den endoskopischen Eingriff zu schaffen.

Um die Einsatzmöglichkeit des solchermaßen ausgestalteten Retraktors weiter zu erhöhen, ist die Ringstruktur darüber hinaus über das Gelenk 5 stufenlos um bis zu 90° aus der Ebene der Ringstruktur abwinkelbar.

Das Gewicht des Handstücks 2 ist so bemessen, daß das Handstück als Gegengewicht zu dem über die Ringstruktur erfaßte Körperteil dient. Hierdurch entfällt das kraftaufwändige Drücken auf das Handstück, wie dies bei den herkömmlichen Retraktoren notwendig ist.

Zum Entfernen des Retraktors aus dem Körper werden die Gliederelemente 4 wieder unter endoskopischer Sichtkontrolle mittels eines Werkzeugs in die gerade gestreckte Ausgangsposition verstellt, so daß der Retraktor wieder problemlos aus dem Körper herausgezogen werden kann.

Nach dem Lösen der Rastverbindung zwischen Schaft 1 und Handstück 2 bzw. Adapterstück 8 kann der Schaft 1 anschließend unabhängig vom Handstück 2 sterilisiert werden.

Neben der dargestellten Ausführungsform eines Retraktors, bei der das Verstellen des distalen Endes des Schaftes 1 unter endoskopischer Sicht mittels eines Werkzeugs erfolgt, wird gemäß einer alternativen Ausführungsform das distale Ende des Schaftes 1 über einen Bowdenzug verstellt. Dieser Bowdenzug ist dann anstelle des Rastelements 6 am vorderen freien Ende des vordersten Gliederelements 4a befestigt und wird durch die Bohrung 7 (Rastaufnahme) in dem Schaft1 zum Handstück 2 geführt. In der gestreckten Position verläuft der Bowdenzug dann zwischen Bohrung 7 und dem freien Ende des vordersten Gliederelements 4a in einer Nut. Für die Streckung des Retraktors wird ein entsprechend auf der Außenseite des Ringes in einer Nut geführter Bowdenzug verwendet.

Neben den hygienischen Gesichtspunkten dieses einfach und gut zu reinigenden Retraktors zeichnet sich dieser Retraktor dadurch aus, daß er einen sicheren atraumatischen Gebrauch gewährleistet.

### Bezugszeichenliste

- 1: Schaft
- 2: Handstück
- 3: starrer Teil
- 4: Gliederelement
- 4a: Gliederelement
- 4b: Gliederelement
- 5: Gelenk
- 6: Rastelement
- 6a: Arm
- 6b: Rastvorsprung
- 6c: Ringnut
- 6d: Stift
- 6e: Kopf
- 7: Rastaufnahme
- 8: Adapterstück
- 9: Durchgangsbohrung
- 10: Rastelement
- 10a: Aufnahmebohrung
- 11: Knopf
- 12: Druckfeder
- 13: Einführhilfe
- 13a: Schaft
- 13b: Spitze
- 14: Ausnehmung

## Patentansprüche

1. Retraktor zur Verwendung in der endoskopischen Chirurgie, mit einem Schaft (1), an dessen proximalem Ende ein Handstück (2) angeordnet ist und dessen distales Ende aus einer gestreckten geraden Ausgangsposition verstellbar ist, wobei das verstellbare distale Ende aus mehreren gegeneinander verschwenkbaren Gliederelementen (4) besteht, die in eine ringförmige Struktur verstellbar sind und wobei diese ringförmige Struktur gegenüber dem starren Teil (3) des Schaft (1) abwinkelbar ist,
**dadurch gekennzeichnet,**
**daß** das distale Ende des Schaftes (1) in eine geschlossene Ringstruktur verstellbar ist, wobei am vorderen freien Ende des vordersten Gliederelements (4a) ein Fixierelement angeordnet ist, über das zum Ausbilden der geschlossenen Ringstruktur das vorderste Gliederelement (4a) am Schaft (1) festlegbar ist, und daß die geschlossene Ringstruktur gegenüber dem starren Teil (3) des Schaftes (1) stufenlos um bis zu 90° abwinkelbar ist.

2. Retraktor nach Anspruch 1, **dadurch gekennzeichnet, daß** das distale Ende des Schaftes (1) unter endoskopischer Sicht mittels eines Werkzeugs in die geschlossene Ringstruktur verstellbar ist.

3. Retraktor nach Anspruch 2, **dadurch gekennzeichnet, daß** am vordersten Gliederelement (4a) Ausnehmungen ausgebildet sind, in die zum Verstellen der Gliederelemente (4a) ein Werkzeug, insbesondere eine Zange, eingreifen kann.

4. Retraktor nach Anspruch 1, **dadurch gekennzeichnet, daß** das distale Ende des Schaftes (1) mittels eines Bowdenzuges in die geschlossene Ringstruktur verstellbar ist.

5. Retraktor nach Anspruch 4, **dadurch gekennzeichnet, daß** das Fixierelement als Rastelement (6) ausgebildet ist, das in einer Rastaufnahme (7) des ersten, mit dem starren Teil (3) des Schaftes (1) verbundenen Gliederelements (4b) festlegbar ist.

6. Retraktor nach Anspruch 4, **dadurch gekennzeichnet, daß** das Fixierelement als Magnet ausgebildet ist.

7. Retraktor nach mindestens einem der Ansprüche 1 bis 6, **dadurch gekennzeichnet, daß** die Ringstruktur über ein Gelenk (5) zwischen dem starren Teil (3) des Schaftes (1) und dem ersten Gliederelement (4b) in beide Richtungen aus der Ebene der Ringstruktur um mindestens bis zu 90° gegenüber dem starren Teil (3) des Schaftes (1) abwinkelbar ist.

8. Retraktor nach Anspruch 1, **dadurch gekennzeichnet, daß** das Handstück (2) und der Schaft (1) auswechselbar über ein Adapterstück (8) miteinander verbunden sind.

9. Retraktor nach Anspruch 8, **dadurch gekennzeichnet, daß** der Schaft (1) mit dem Adapterstück (8) verrastet ist.

10. Retraktor nach Anspruch 8 oder 9, **dadurch gekennzeichnet, daß** das Adapterstück (8) eine axiale Bohrung (9) aufweist, in der ein von außen über einen Knopf (11) betätigbares Rastelement (10) zum Festlegen des Schaftes (1) angeordnet ist.

11. Retraktor nach Anspruch 10, **dadurch gekennzeichnet, daß** das Rastelement (10) in der Raststellung federbelastet ist.

12. Retraktor nach mindestens einem der Ansprüche 1 bis 11, **dadurch gekennzeichnet, daß** das Handstück (2) als Gegengewicht zu dem mittels der Ringstruktur erfaßten Körperteils dient.

## Claims

1. Retractor for use in endoscopic surgery, with a shaft (1) at whose proximal end a handpiece (2) is arranged and whose distal end can be adjusted from an extended straight starting position, the adjustable distal end consisting of several mutually pivotable link elements (4) which can be adjusted into a ring-shaped structure, and this ring-shaped structure being able to be bent at an angle in relation to the rigid part (3) of the shaft (1), **characterized in that** the distal end of the shaft (1) can be adjusted into a closed ring structure, a fixing element being arranged at the forward free end of the frontmost link element (4a), via which fixing element the frontmost link element (4a) can be secured on the shaft (1) in order to form the closed ring structure, and **in that** the closed ring structure can be bent steplessly to an angle of up to 90° in relation to the rigid part (3) of the shaft (1).

2. Retractor according to Claim 1, **characterized in that** the distal end of the shaft (1) can be adjusted into the closed ring structure, under endoscopic monitoring, by means of a tool.

3. Retractor according to Claim 2, **characterized in that**, on the frontmost link element (4a), there are recesses into which a tool, in particular a pincer, can engage so as to adjust the link elements (4a).

4. Retractor according to Claim 1, **characterized in that** the distal end of the shaft (1) can be adjusted into the closed ring structure by means of a Bowden cable.

5. Retractor according to Claim 4, **characterized in that** the fixing element is designed as a catch element (6) which can be secured in a catch receiver (7) of the first link element (4b) which is connected to the rigid part (3) of the shaft (1) .

6. Retractor according to Claim 4, **characterized in that** the fixing element is designed as a magnet.

7. Retractor according to at least one of Claims 1 to 6, **characterized in that** the ring structure can be set to an angle of up to at least 90° in relation to the rigid part (3) of the shaft (1), in both directions from the plane of the ring structure, by means of a hinge (5) between the rigid part (3) of the shaft (1) and the first link element (4b) .

8. Retractor according to Claim 1, **characterized in that** the handpiece (2) and the shaft (1) are connected to one another detachably via an adapter piece (8) .

9. Retractor according to Claim 8, **characterized in that** the shaft (1) is locked onto the adapter piece (8).

10. Retractor according to Claim 8 or 9, **characterized in that** the adapter piece (8) has an axial bore (9) in which a catch element (10) activated from outside via a button (11) is arranged for securing the shaft (1).

11. Retractor according to Claim 10, **characterized in that** the catch element (10) is spring-loaded in the catch position.

12. Retractor according to at least one of Claims 1 to 11, **characterized in that** the handpiece (2) serves as a counterweight to the body part gripped by means of the ring structure.

## Revendications

1. Rétracteur destiné à être utilisé en chirurgie endoscopique, comportant un fût (1), à l'extrémité proximale duquel est agencée une pièce à main (2), et dont l'extrémité distale duquel est réglable depuis une position initiale droite en extension, l'extrémité distale réglable étant constituée de plusieurs éléments de maillons (4) susceptibles de pivoter les uns par rapport aux autres, qui sont réglables pour former une structure annulaire, et cette structure annulaire étant susceptible d'être coudée par rapport à la partie rigide (3) du fût (1),
**caractérisé en ce que**
l'extrémité distale du fût (1) est réglable pour former une structure annulaire fermée, un élément de fixation étant agencé sur l'extrémité libre antérieure de l'élément de maillon (4a) situé le plus en avant, élément de fixation via lequel on peut immobiliser l'élément de maillon (4a) situé le plus en avant sur le fût (1) pour former la structure annulaire fermée, et **en ce que** la structure annulaire fermée peut être coudée en continu jusqu'à 90° par rapport à la partie rigide (3) du fût (1).

2. Rétracteur selon la revendication 1, **caractérisé en ce que** l'extrémité distale du fût (1) est réglable sous observation endoscopique au moyen d'un outil pour former la structure annulaire fermée.

3. Rétracteur selon la revendication 2, **caractérisé en ce que** sur l'élément de maillon (4a) situé le plus en avant sont réalisés des évidements dans lesquels peut s'engager un outil, en particulier une pince pour régler les éléments de maillons (4a).

4. Rétracteur selon la revendication 1, **caractérisé en ce que** l'extrémité distale du fût peut être réglée pour former la structure annulaire fermée au moyen d'un câble Bowden.

5. Rétracteur selon la revendication 4, **caractérisé en ce que** l'élément de fixation est réalisé sous forme d'élément d'enclenchement (6) qui peut être immobilisé dans un logement d'enclenchement (7) du premier élément de maillon (4b) relié à la partie rigide (3) du fût (1).

6. Rétracteur selon la revendication 4, **caractérisé en ce que** l'élément de fixation est réalisé sous forme d'aimant.

7. Rétracteur selon au moins l'une des revendications 1 à 6, **caractérisé en ce que** la structure annulaire peut être coudée d'au moins jusqu'à 90° par rapport à la partie rigide (3) du fût (1) dans les deux directions depuis le plan de la structure annulaire, via une articulation (5) entre la partie rigide (3) du fût (1) et le premier élément de maillon (4b).

8. Rétracteur selon la revendication 1, **caractérisé en ce que** la pièce à main (2) et le fût (1) sont reliés mutuellement via un adaptateur (8) de manière à pouvoir être remplacés.

9. Rétracteur selon la revendication 8, **caractérisé en ce que** le fût (1) est enclenché avec l'adaptateur (8).

10. Rétracteur selon l'une ou l'autre des revendications 8 et 9, **caractérisé en ce que** l'adaptateur (8) comporte un perçage axial (9) dans lequel est agencé un élément d'enclenchement (10) actionnable depuis l'extérieur par un bouton (11) pour immobiliser le fût (1).

11. Rétracteur selon la revendication 10, **caractérisé en ce que** l'élément d'enclenchement (10) est sollicité par ressort dans la position d'enclenchement.

12. Rétracteur selon au moins l'une des revendications 1 à 11, **caractérisé en ce que** la pièce à main (2) sert de contrepoids à la partie du corps saisie au moyen de la structure annulaire.
